# EUROPÄISCHE PATENTANMELDUNG

(11) **EP 1 329 148 A1**
(43) Veröffentlichungstag der Anmeldung: **23.07.2003**
(21) Anmeldenummer: 02102760.2
(22) Anmeldetag: 16.12.2002
(51) Int. Cl.: A01B 79/00

(54) **Roboter zum Beproben und Analysieren des Erdbodens**

(30) Priorität: 19.12.2001 US 24907
(71) Anmelder: DEERE & COMPANY, Moline, Illinois 61265-8098 (US)
(72) Erfinder: Pickett, Terence, D., IA 50263, Waukee (US)
(74) Vertreter: Holst, Sönke, Dr.

(57) **Zusammenfassung**

Die Erfindung bezieht sich auf einen Roboter (10) zum Beproben und Analysieren des Erdbodens, mit einem Bodenvortriebssystem (14) zum Bewegen des Roboters über den Erdboden, einer Steuerungseinheit (16) zur Steuerung des Bodenvortriebssystems (14), einer Sonde (20) zur Entnahme einer Bodenprobe, einem Labor (24) zur Analyse der Bodenprobe, einem Prozessor (26) zur Erzeugung von Daten von der Bodenanalyse, und einem Sender (28) zum Übertragen der Daten an einen beabstandeten Ort.

## Beschreibung

Die Erfindung betrifft einen Roboter zum Beproben und Analysieren des Erdbodens.

Bodentests sind gebräuchlich und in der Landwirtschaft erforderlich, um den Bodentyp und den Nährstoffgehalt festzustellen, so dass die Pflanzenproduktion maximiert werden kann. Typischerweise erfordern Bodentests, dass Bodenproben oder -muster im Feld entnommen werden und dann zur Laboranalyse fortgeschickt werden. Normalerweise bedient eine Person die Bodenprobenentnahmemaschine oder das -fahrzeug und schreibt die Stelle auf, an der die Proben entnommen wurden. Dieser herkömmliche Testprozess ist wegen der Arbeitskraftanforderungen zum Betrieb der Maschine und der Verzögerungen beim Transferieren der Proben vom getesteten Gelände zum davon entfernten Labor zwecks der Analyse zeitaufwändig und teuer.

Die der Erfindung zu Grunde liegende Aufgabe wird darin gesehen, ein automatisches System zum Sammeln und Analysieren von Bodenproben auf einem Feld bereitzustellen.

Diese Aufgabe wird erfindungsgemäß durch die Lehre der Patentansprüche 1 und 5 gelöst, wobei in den weiteren Patentansprüchen Merkmale aufgeführt sind, die die Lösung in vorteilhafter Weise weiterentwickeln.

Die Erfindung schlägt einen Roboter vor, der zur Entnahme und Analyse von Bodenproben auf einem Feld verwendbar ist. Der Roboter umfasst ein Bodenvortriebssystem, um ihn über den Boden zu bewegen und eine z. B. mit einem satellitengestützten Ortungssystem zusammenwirkende Steuerungseinheit zur Steuerung des Bodenvortriebssystems und zum Lenken des Roboters. Der Roboter umfasst außerdem eine Sonde zur Entnahme von Bodenproben und ein Labor zur Analyse der Bodenproben. Ein Prozessor des Roboters ist bereitgestellt, um Daten von der Bodenanalyse zu erzeugen, und ein Sender überträgt dann die Daten an einen beabstandeten Ort. Es wäre auch denkbar, anstelle oder zusätzlich zu der Übertragung die Daten auf dem Roboter auf einem Datenträger abzuspeichern, um den Datenträger später auswerten zu können.

Auf diese Weise werden alle genannten Schritte selbsttätig durchgeführt. Eine Beprobung eines Felds kann schneller und kostengünstiger durchgeführt werden.

In den Zeichnungen ist ein nachfolgend näher beschriebenes Ausführungsbeispiel der Erfindung dargestellt. Es zeigt:
- Fig. 1: ein Blockdiagramm, in dem die verschiedenen Module dargestellt sind, die das erfindungsgemäße Roboterfahrzeug betreiben,
- Fig. 2: eine schematische perspektivische Ansicht des Roboterfahrzeugs.

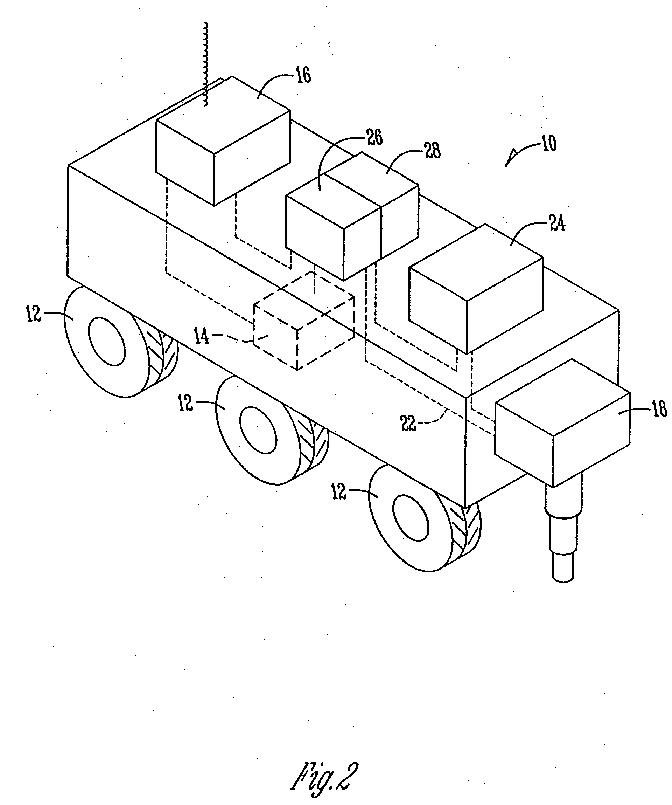

Die vorliegende Erfindung bezieht sich auf ein Verfahren und ein Roboterfahrzeug zur Beprobung und Analyse von Erdboden. Das Roboterfahrzeug oder die Plattform ist in Figur 2 insgesamt mit dem Bezugszeichen 10 gekennzeichnet. Der Roboter 10 umfasst eine Mehrzahl an Rädern 12 oder anderen Vortriebsmitteln, die drehbar durch ein Bodenvortriebssystem 14 angetrieben werden, das einen Diesel-, Benzin-, Elektro- oder Hybridmotor umfassen könnte. Das Bodenvortriebssystem 14 wird durch eine Steuerungseinheit 16 gesteuert. Die Steuerungseinheit 16 umfasst ein Positionsermittlungssystem zum Empfang von Signalen des Global Positioning Systems (GPS), das auch in Kombination mit anderen Ortsbestimmungstechnologien genutzt werden könnte.

Der Roboter 10 umfasst ein Werkzeugpaket 18, das eine oder mehrere Bodensonden 20 umfasst. Die Sonden 20 können jeglichen konventionellen Aufbaus sein, so dass sie in den Boden eingebracht werden können, um eine Probe oder ein Muster davon zu entnehmen. Es ist anzumerken, dass das Werkzeugpaket 18 mit anderen Werkzeugpaketen vertauscht werden kann, einschließlich verschiedener Sonden 20, wie sie für verschiedene Bodentypen oder Analyseverfahren erforderlich sind.

Jede von der Sonde 20 entnommene Bodenprobe wird durch einen Förderer 22 in ein Labor 24 auf dem Roboter 10 gefördert. Das Labor 24, vorzugsweise ein miniaturisiertes Nasslabor, vollzieht selbsttätig eine Analyse der Bodenproben.

Ein programmierter Prozessor 26 steht mit dem Labor 24 in Betriebsverbindung, um die Bodenprobenanalyse betreffende Daten zu generieren. Der Prozessor 26 umfasst außerdem Wegplanungssoftware, die mit der Steuerungseinheit 16 zur Navigation des Roboters 10 auf dem Feld betreibbar ist. Ein mit dem Prozessor 26 in Betriebsverbindung stehender Sender 28, der auch bidirektional arbeiten und somit Befehle für den Betrieb des Roboters 10 erhalten kann, z. B. bezüglich der Probenentnahmeorte oder der zu verwendenden Analyseverfahren, sendet dann durch Radiofrequenzwellen die Daten an einen vom Feld beabstandeten Ort zwecks Abspeicherns und späterer Verwendung. Die Bodendaten können nach Analyse jeder Probe, d. h. einzeln, oder zwischengespeichert und nach Aufnahme mehrerer Proben, z. B. nach Ende eines Auftrags, übertragen werden.

Der Betrieb des Werkzeugpakets 18 einschließlich der Sonde 20 wird durch den Prozessor 26 gesteuert. In ähnlicher Weise steuert der Prozessor 26 die Aktivierung und Deaktivierung des Bodenvortriebssystems 14.

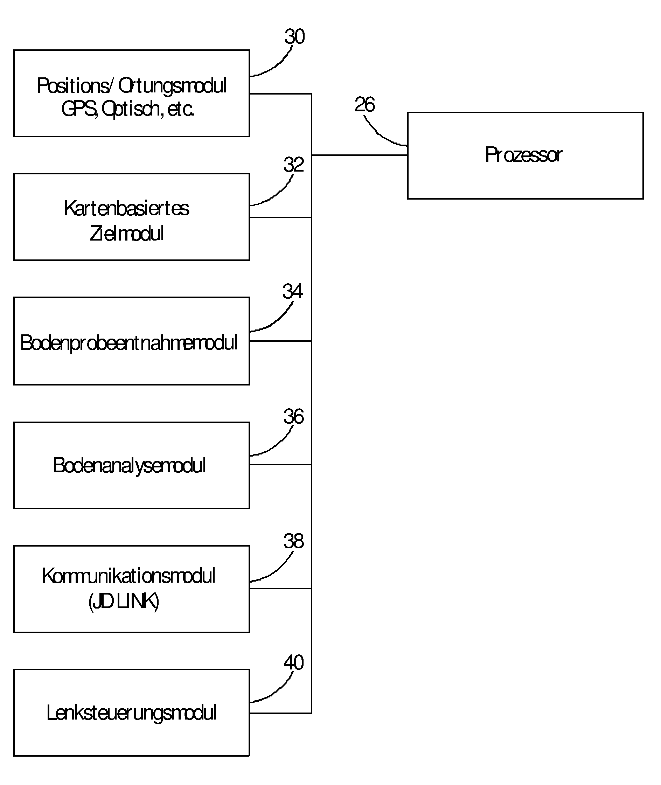

Das Softwareprogramm für den Prozessor 26 umfasst verschiedene Module, wie in Figur 1 dargestellt. Im Einzelnen ist ein erstes Modul 30 bereitgestellt für das Zusammenspiel zwischen dem Prozessor 26 und der Steuerungseinrichtung 16 zur Feststellung der Position und des Orts unter Verwendung des GPS oder einer anderen, z. B. optischen, Ortsbestimmungstechnologie. Ein zweites Modul 32 stellt ein kartenbasiertes Ziel bereit, um eine Entnahme der Bodenproben an gewünschten Orten zu erlauben. Das Bodenprobenmodul 34 wirkt mit dem Prozessor 26 und dem Werkzeugpaket 18 zusammen, um die Probenentnahme aus dem Boden zu steuern. Ein Bodenanalysemodul 36 wirkt mit dem Prozessor 26 und dem Labor 24 zusammen, um die Bodendaten zu generieren. Ein Kommunikationsmodul 38 wirkt mit dem Prozessor 26 und dem Sender 28 zusammen, so dass die Bodendaten an den beabstandeten Ort übertragen werden können. Ein Lenksteuerungsmodul 40 wirkt mit dem Prozessor 26 und dem Bodenvortriebssystem 40 zusammen, um den Roboter 10 auf dem Feld zu lenken.

Das erfindungsgemäße Verfahren zum automatischen Bodenbeproben und -analysieren umfasst die Schritte des Bewegens des Roboters 10 über den Erdboden und ein Entnehmen einer oder mehrerer Bodenproben unter Verwendung der Bodensonde oder Bodensonden 20 des Werkzeugpakets 18. Die Bodenprobe oder Bodenproben werden durch den Förderer 22 in das Labor 24 gefördert und darin analysiert. Daten von der Bodenanalyse werden durch den Prozessor 26 erzeugt und durch den Sender 28 an den beabstandeten Ort übertragen. Der gesamte Probenentnahme- und Analysebetrieb, einschließlich der Datenerzeugung und Übertragung, wird durch den unbemannten Roboter 10 autonom durchgeführt, ohne menschlichen Eingriff. Es gibt daher Kostenersparnisse im Vergleich mit bekannten, bemannten Bodenentnahmeverfahren.

Die Kommunikationsfähigkeiten des Roboters 10 erlauben es dem Roboter, den Bodenbeprobungs- und -analyseauftrag in einer begrenzten Fläche auszuführen und dann nach Vollenden der Operation zu warten, bis er aufgenommen und abtransportiert wird.

Aus dem Vorhergehenden ist erkennbar, dass das erfindungsgemäße Gerät und Verfahren die Kosten für die Bodenanalyse wesentlich vermindern und es ermöglichen, die Anzahl der analysierten Proben zu vergrößern, um die Auflösung der gewonnenen Daten zu verbessern. Zusätzliche Werkzeugpakete können dem Roboter 10 zugefügt werden, um den Typ und den Grad eines Schädlings- oder Pilzbefalls feststellen zu können und es dem Produzenten zu ermöglichen, schneller und mit einem gezielteren Ansatz gegen derartige Schädlinge vorgehen zu können.

## Patentansprüche

1. Verfahren zur automatischen Bodenbeprobung und Analyse, mit folgenden Schritten:
Bewegen eines Roboters (10) über den Erdboden,
Entnehmen einer Bodenprobe unter Verwendung einer Bodensonde (20) des Roboters (10),
Analysieren der Bodenprobe in einem Labor (24) des Roboters (10),
Erzeugen von Daten der Bodenprobenanalyse, und
Übertragen der Daten an einen beabstandeten Ort.

2. Verfahren nach Anspruch 1, **dadurch gekennzeichnet, dass** die Bewegung des Roboters (10) mit einem globalen Positionierungssystem oder einem anderen Ortungssystem oder einer Kombination verschiedener Ortungssysteme gesteuert wird.

3. Verfahren nach Anspruch 1 oder 2, **dadurch gekennzeichnet, dass** es ohne menschliche Einwirkung durchgeführt wird.

4. Verfahren nach einem der Ansprüche 1 bis 3, **dadurch gekennzeichnet, dass** der Roboter (10) die Schritte autonom durchführt.

5. Roboter (10) zum Beproben und Analysieren des Erdbodens, mit:
einem Bodenvortriebssystem (14) zum Bewegen des Roboters über den Erdboden,
einer Steuerungseinheit (16) zur Steuerung des Bodenvortriebssystems (14),
einer Sonde (20) zur Entnahme einer Bodenprobe,
einem Labor (24) zur Analyse der Bodenprobe,
einem Prozessor (26) zur Erzeugung von Daten von der Bodenanalyse, und
einem Sender (28) zum Übertragen der Daten an einen beabstandeten Ort.

6. Roboter (10) nach Anspruch 5, **dadurch gekennzeichnet, dass** die Steuerungseinheit (16) mit einem globalen Positionierungssystem zusammenwirkt.

7. Roboter (10) nach Anspruch 5 oder 6, **dadurch gekennzeichnet, dass** er unbemannt ist.

8. Roboter (10) nach einem der Ansprüche 5 bis 7, **dadurch gekennzeichnet, dass** die Steuerungseinheit (16) den Roboter (10) lenkt.

9. Roboter (10) nach einem der Ansprüche 5 bis 8, **dadurch gekennzeichnet, dass** er einen Förderer (18) umfasst, um die Bodenprobe in das Labor (24) zu fördern.

10. Roboter (10) nach einem der Ansprüche 5 bis 9, **dadurch gekennzeichnet, dass** der Prozessor (26) mit dem Bodenvortriebssystem (14) in Betriebsverbindung steht, um es zu aktivieren und zu deaktivieren.

11. Roboter (10) nach einem der Ansprüche 5 bis 10, **dadurch gekennzeichnet, dass** der Prozessor (26) mit der Steuerungseinheit (16) in Betriebsverbindung steht, um den Roboter (10) selbsttätig zu bewegen.

12. Roboter (10) nach einem der Ansprüche 5 bis 11, **dadurch gekennzeichnet, dass** der Prozessor (26) zur automatischen Analyse der Bodenprobe mit dem Labor (24) in Betriebsverbindung steht.

13. Roboter (10) nach einem der Ansprüche 5 bis 12, **dadurch gekennzeichnet, dass** der Prozessor (26) mit dem Sender (28) zur automatischen Übertragung der Daten in Betriebsverbindung steht.

14. Roboter (10) nach einem der Ansprüche 5 bis 13, **dadurch gekennzeichnet, dass** der Sender (28) Radiofrequenz oder Mobilfunktechnologie verwendet, um die Daten zu übertragen.
